# EUROPEAN PATENT APPLICATION

(11) **EP 4 249 003 A1**
(43) Date of publication of application: **27.09.2023**
(21) Application number: 21894725.7
(22) Date of filing: 18.11.2021
(51) Int. Cl.: A61K 48/00, A61K 35/76, A61K 35/761, A61K 38/22, A61P 1/18, A61P 3/10, A61P 43/00, C12N 15/12, C12N 15/86, C12N 15/861

(54) **LOW-DOSE HEPATOCYTE GROWTH FACTOR GENE THERAPY FOR DIABETES**

(30) Priority: 19.11.2020 JP 2020192844; 07.09.2021 JP 2021145795
(71) Applicant: Kagoshima University, Kagoshima-shi, Kagoshima 890-8580 (JP)
(72) Inventor: KOSAI, Ken-ichiro, Kagoshima-shi, Kagoshima 890-8580 (JP); MATSUDA, Eriko, Kagoshima-shi, Kagoshima 890-8580 (JP)
(74) Representative: Weickmann & Weickmann PartmbB
(86) International application number: PCT/JP2021/042463
(87) International publication number: WO 2022/107853

(57) **Abstract**

An agent for protecting and/or regenerating pancreatic β cells in a mammal with diabetes, containing a recombinant viral vector expressing a hepatocyte growth factor (HGF), wherein the agent is administered at a dose of 10¹⁰ - 10¹² virus particles (vp)/kg body weight, and the viral vector contains a nucleic acid encoding HGF downstream of a promoter with transcriptional activity capable of affording a therapeutically effective blood HGF level at said dose is provided by the present invention.

## Description

### [Technical Field]

The present invention relates to a gene therapeutic agent for diabetes, that simultaneously achieves safety and therapeutic effects. More particularly, the present invention relates to a gene therapeutic agent for diabetes, which is characterized in that it contains a viral vector containing a nucleic acid encoding a hepatocyte growth factor (HGF) downstream of a promoter capable of expressing a therapeutically effective amount of HGF, and is administered at a low dose, and which does not substantially express adverse events due to the viral vector.

### [Background Art]

According to a report by the International Diabetes Federation (IDF), the number of diabetes patients in the world has reached 463 million as of 2019, and is predicted to reach 700 million by 2045 if the number continues to grow at this rate. In type 2 diabetes (T2D) accounting for 90% of all diabetes, when insulin sensitivity decreases due to genetic predisposition and environmental factors (lifestyle), pancreatic β cells try to compensate for the lack of insulin action by compensatory hypersecretion of insulin. If this state continues for a long time, the β cells become exhausted, and cannot secrete a sufficient amount of insulin, resulting in hyperglycemia.

On the other hand, type 1 diabetes (T1D) is a disease in which insulin secretion is depleted due to destruction of pancreatic β cells by an autoimmune mechanism, leading to hyperglycemia. T1D develops at a young age and the details of its mechanism are still unclear.

T1D patients need to self-inject insulin for the rest of their lives to control their blood sugar. However, glycemic control is difficult even when strict insulin therapy is performed, and hyperglycemia and severe hypoglycemia recur, making it difficult to prevent progress of complications. Therefore, an innovative therapeutic method that can completely cure T1D is demanded.

As a therapeutic method that completely cures T1D, expectations are rising for regenerative medicine that regenerates and reconstructs lost organ functions. One of them is β cell supplementation therapy by pancreatic islet transplantation, and this treatment method has been reported to be effective in improving glucose metabolism. However, since T1D patients must use immunosuppressants after pancreatic islet transplantation and there is a shortage of donors for pancreatic islet transplantation, the practical application of this technique is limited.

As another approach of regenerative medicine for T1D, therefore, attempts have been made to directly "protect (anti-cell death induction) and/or proliferate (regenerate) (regenerative healing induction)" (hereinafter sometimes to be comprehensively referred to as "protecting and/or regenerating") the patient's own remaining β cells in the body by in vivo gene therapy. For example, some studies using hepatocyte growth factor (HGF) as a therapeutic gene for T1D have been reported (Non Patent Literatures 1-3). HGF was first identified as a potent hepatocyte mitogen, but is now known to be a multifunctional cytokine, and exerts therapeutic effects such as cytoprotection, anti-fibrosis, and induction of regeneration in many diseases in preclinical and clinical studies. In the pancreas, HGF is expressed in endothelial cells and mesenchymal cells, and the HGF receptor c-Met is also known to be localized in pancreatic progenitor cells, pancreatic islet cells, and pancreatic ductal cells.

Non Patent Literature 1 discloses that a plasmid (20 µg) containing a nucleic acid encoding HGF was administered to mice before the onset of diabetes to maintain insulin secretion. Non Patent Literature 2 discloses that an adeno-associated virus (AAV) vector containing a nucleic acid encoding HGF was administered at a high dose (3.0×10¹¹ vector genome (vg); about 1.5×10¹³ vg per kg body weight) to mice before the onset of diabetes, through the pancreatic duct to suppress the rise in blood sugar. However, in both cases, the HGF-expressing vector was administered "before" the onset of T1D, and therefore, its usefulness in clinical application to T1D patients with many already-destroyed β-cells has not been shown. Furthermore, the latter intraductal administration is highly invasive, although gene transfer is mostly confined to the pancreas, and is not realistic as a means of administration in human clinical situations.

Non Patent Literature 3 reports that administration of a high dose (1.0×10¹¹ virus particles (vp); about 5×10¹² vp per kg body weight) of an adenovirus (Ad) vector expressing HGF from the tail vein of mice after the onset of T1D resulted in a partial success.

However, in 1999, in a clinical trial at the University of Pennsylvania for ornithine transcarbamylase deficiency, which is an inborn metabolic disease, a fatal accident occurred due to a systemic inflammatory reaction caused by hepatic artery administration of 6×10¹¹ vp/kg body weight (the total amount 3.8×10¹³ vp) of Ad vector (Non Patent Literature 4). Therefore, clinical application of high doses of Ad vectors has not been performed since then in gene therapy involving systemic administration of Ad from blood vessels in vivo, regardless of the target disease. Since such risk was considered to be limited to Ad, AAV vector has been generally used in clinical application of in vivo gene therapy (systemic administration from blood vessels) for congenital diseases and the like since then to now.

Recently (June 23, 2020), however, even in AAV vectors that have long been believed to be apathogenic and highly safe, three cases of serious adverse events, particularly death due to severe liver damage, were reported in a clinical trial of gene therapy for congenital myopathy, in patients who received high dose (3×10¹⁴ vg/kg) of AAV vectors (Non Patent Literature 5, Non Patent Literature 6). As described above, AAV vectors are currently the most widely used vectors for clinical applications of in vivo gene therapy (systemic administration from blood vessels). However, this report has just clarified that systemic administration of high doses of vectors from blood vessels in vivo possibly causes serious adverse events including dangerous liver damage, regardless of the type of vector. The clinical application of in vivo gene therapy with high dose vectors has been discontinued and reviewed.

As described, fatal accidents caused by AAV vectors have become major global and historical events in the field of gene therapy.

### [Citation List]

### [Non Patent Literature]

[NPL 1]
   Dai C, Li Y, Yang J et al. Hepatocyte growth factor preserves beta cell mass and mitigates hyperglycemia in streptozotocininduced diabetic mice. J Biol Chem 2003; 278: 27080-27087.
[NPL 2]
   Jimenez V, Ayuso E, Mallol C et al. In vivo genetic engineering of murine pancreatic beta cells mediated by single-stranded adeno-associated viral vectors of serotypes 6, 8 and 9. Diabetologia 2011; 54: 1075-1086.
[NPL 3]
   Park MK, Kim DK, Lee HJ. Adenoviral mediated hepatocyte growth factor gene attenuates hyperglycemia and beta cell destruction in overt diabetic mice. Exp Mol Med 2003; 35: 494-500.
[NPL 4]
   Raper SE, Chirmule N, Lee FS et al. Fatal systemic inflammatory response syndrome in a ornithine transcarbamylase deficient patient following adenoviral gene transfer. Mol Genet Metab 2003; 80: 148-158.
[NPL 5]
   Audentes Therapeutics. Letter to the MTM disease community. https://myotubulartrust.org/audentes-therapeutics-letter-23-june-2020/.
[NPL 6]
   Wilson JM, Flotte TR. Moving Forward After Two Deaths in a Gene Therapy Trial of Myotubular Myopathy. Hum Gene Ther 2020; 31: 695-696.

### [Summary of Invention]

### [Technical Problem]

Therefore, the present invention aims to provide a gene therapy means for diabetes including T1D, that has simultaneously achieved safety and therapeutic effects.

### [Solution to Problem]

In order to solve the above-mentioned problems, the present inventors first took note of the HGF gene as a therapeutic gene. In all of the HGF gene therapies for T1D reported so far, HGF is expressed under the transcriptional control of the cytomegalovirus (CMV) promoter (the above-mentioned Non Patent Literatures 1-3), and viral vectors are administered at high doses of about 5×10¹² to about 1.5×10¹³ vp/kg body weight. The present inventors have considered that it is necessary to increase the expression efficiency of therapeutic genes in order to simultaneously achieve the desired therapeutic effect while realizing a low dose of viral vectors to ensure the safety of in vivo gene therapy. Therefore, they produced an Ad vector that expresses HGF under the transcriptional control of the CA promoter (hybrid promoter of CMV-immediate-early enhancer and modified chicken β-actin promoter; also called "CAG promoter", but collectively denoted as "CA promoter" in the present specification), which has been reported to exhibit stronger transcriptional activity in various cell types than the CMV promoter and other widely used ubiquitous promoters, and administered same to model mice after the onset of T1D through the tail vein at a dose one to two orders of magnitude lower than previously reported (3×10⁸ pfu; corresponding to 10⁹ - 10¹⁰ vp). As a result, in the HGF-treated group, blood glucose elevation was significantly suppressed for about 1 week after administration as compared to the non-treated group, and the suppression tendency was maintained for a long period thereafter (at least 11 weeks after administration). In the non-treated group, compensatory hypersecretion of insulin from the remaining β cells was observed in the acute phase of T1D, whereas in the HGF-treated group, normal insulin secretion was maintained. Also, in the IPGTT, the blood glucose elevation was significantly suppressed and the insulin secretion level was significantly maintained on the day 16 and day 60 after administration. In the below-mentioned Examples, even though an Ad vector for transient expression (generally 2 to 3 weeks) was used for gene transfer, a hyperglycemia suppressing effect and a glucose tolerance improving effect were surprisingly sustained successfully far beyond the predicted expression period.

These results show that even with low dose viral vector administration, HGF had the effect of protecting and/or regenerating β cells, and that the effect was sustained for a long period far beyond the expectation. On the other hand, no hepatopathy due to viral vector administration was observed, no changes in appetite, exercise, and the like were observed during the subsequent observation period, and no adverse events were observed.

Based on these findings, the present inventors have succeeded in treating T1D safely and effectively by administering a viral vector that expresses HGF under the transcriptional control of a promoter capable of expressing a therapeutically effective amount of HGF, at a dose lower than the existing dose, and completed the present invention.

Accordingly, the present invention provides the following.
[1] An agent for protecting and/or regenerating pancreatic β cells in a mammal with diabetes, comprising a recombinant viral vector expressing a hepatocyte growth factor (HGF), wherein the agent is administered at a dose of 10¹⁰ - 10¹² virus particles (vp)/kg body weight, and the viral vector comprises a nucleic acid encoding HGF downstream of a promoter with transcriptional activity capable of affording a therapeutically effective blood HGF level at said dose.
[2] The agent of [1], wherein the aforementioned viral vector is an adenovirus (Ad) vector or an adeno-associated virus (AAV) vector.
[3] The agent of [2], wherein the agent is administered in a single dose or multiple doses at an administration interval of at least 60 days.
[4] The agent of any of [1] to [3], wherein the aforementioned promoter is a CA promoter.
[5] The agent of any of [1] to [4], wherein the diabetes is type 1 diabetes.
[6] The agent of any of [1] to [5], wherein the mammal is human.
[7] The agent of any of [1] to [6], wherein the agent is administered by systemic administration.
[8] The agent of [7], wherein the systemic administration is intravenous administration.
[9] The agent of [8], wherein the intravenous administration is administration via a peripheral vein.
[10] A method for protecting and/or regenerating pancreatic β cells in a mammal with diabetes, comprising administering a recombinant viral vector expressing HGF to the mammal, wherein the viral vector is administered at a dose of 10¹⁰ - 10¹² virus particles (vp)/kg body weight, and comprises a nucleic acid encoding HGF downstream of a promoter with transcriptional activity capable of affording a therapeutically effective blood HGF level at said dose.

### [Advantageous Effects of Invention]

According to the present invention, the desired effect of protecting and/or regenerating pancreatic β cells can be achieved over a long period of time even with administration at low doses. Accordingly, in vivo high QOL gene therapy using HGF for diabetes that requires insulin administration, including T1D, becomes possible while ensuring safety, and the realizability of the application of this treatment to human clinical practice can be enhanced.

### [Brief Description of Drawings]

[Fig. 1]

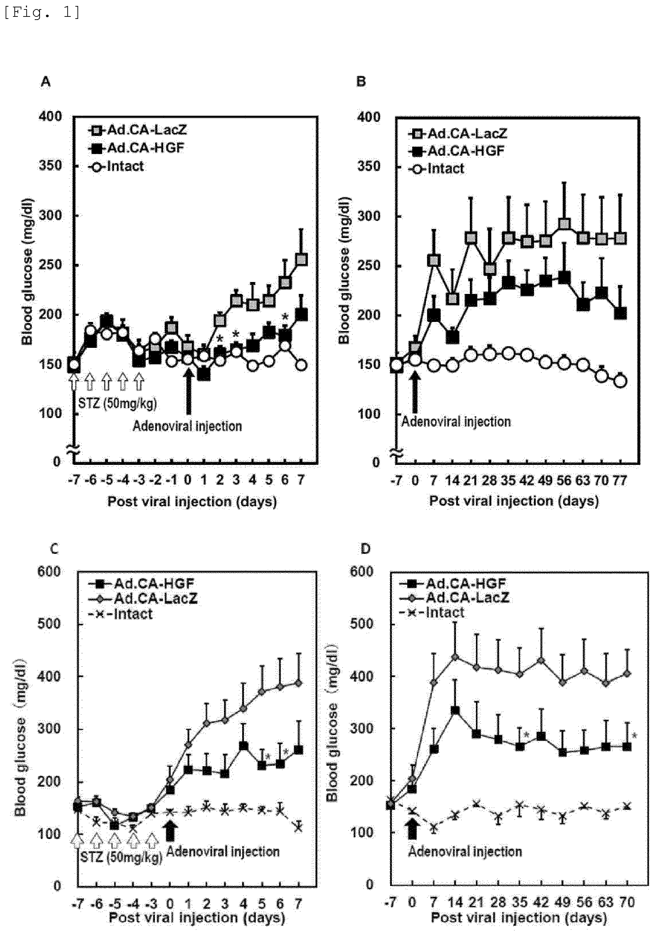

   Figs. 1A and 1C show, in two independent experiments, daily changes in blood glucose level between day -7 to day 7 when adenovirus vector was injected from the tail vein to mice that developed T1D by streptozotocin (STZ) administration. The day when the adenovirus vector was administered is Day 0. Figs. 1B and 1D show weekly changes in blood glucose level between day -7 to day 77 (or 70) in the aforementioned T1D model mice administered with the adenovirus vector (Fig. 1B corresponds to Fig. 1A, Fig. 1D corresponds to Fig. 1C). In the figure, "Ad.CA-HGF" is the viral vector of the present invention that expresses HGF gene under the control of CA promoter, "Ad.CA-LacZ" is a control viral vector with the β-galactosidase gene (LacZ) inserted instead of HGF, and "Intact" shows data from normal mice that did not receive any treatment. In each figure, the horizontal axis indicates the number of days (days) after viral vector administration, and the vertical axis indicates the blood glucose level (mg/dl). *: p<0.05
[Fig. 2]

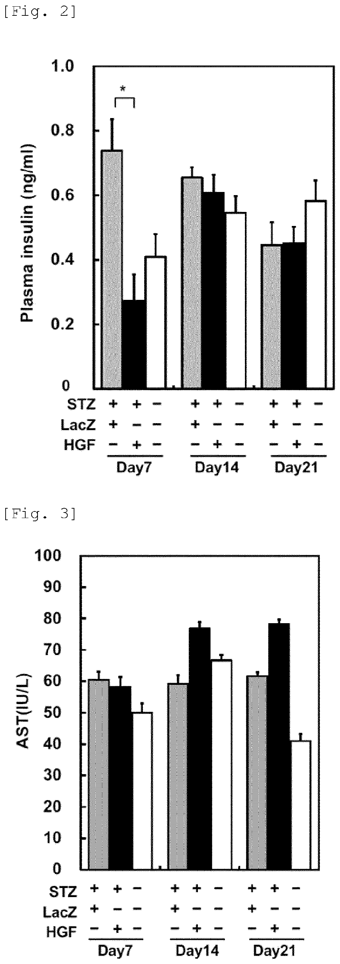

   Fig. 2 shows the measurement results of plasma insulin concentration on day 7, day 14, and day 21 in the same mice as in Fig. 1 (gray bar: T1D model mouse administered with Ad.CA-LacZ; black bar: T1D model mouse administered with Ad.CA-HGF; white bar: normal mice free of STZ administration or viral vector administration). The vertical axis indicates plasma insulin level (ng/ml). *: p<0.05
[Fig. 3]
   Fig. 3 shows the measurement results of plasma AST level on day 7, day 14, and day 21 in the same mice as in Fig. 1 (each bar is as defined in Fig. 2). The vertical axis indicates plasma AST level (IU/L).
[Fig. 4]

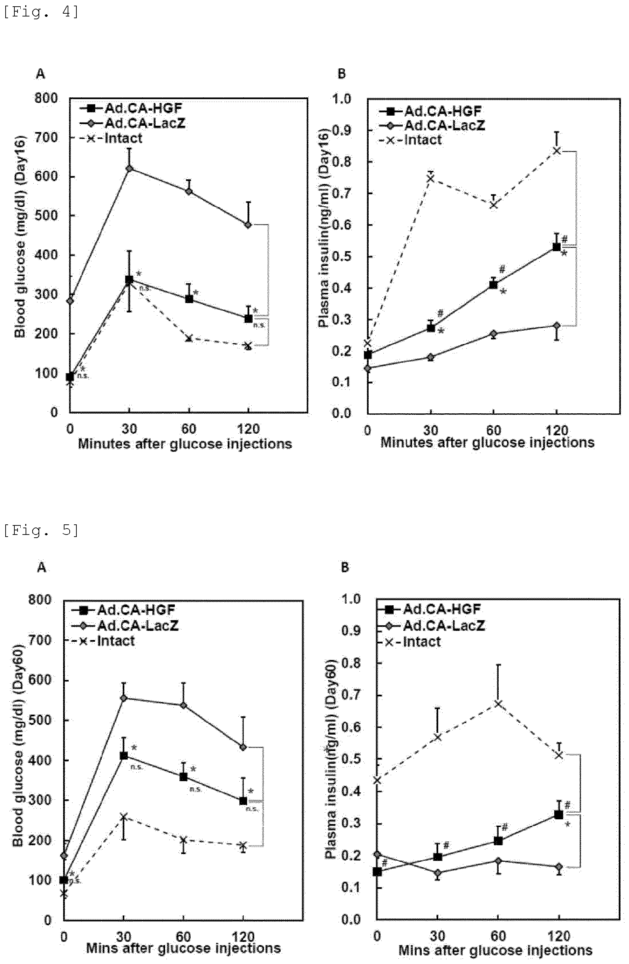

   Fig. 4 shows the results of IPGTT in the same mice as in Fig. 1 (16 days after Ad vector administration). Blood was collected at the time when glucose (2 g/kg body weight) was administered, 30, 60 and 120 min after administration, and blood glucose level and plasma insulin level were measured. Fig. 4A shows time-course changes in the blood glucose level (mg/dl) after glucose administration, and Fig. 4B shows time-course changes in the plasma insulin level (ng/ml) after glucose administration. *: p<0.05 (with respect to Ad.CA-LacZ); #: p<0.05 (with respect to Intact); n.s.: not significant
[Fig. 5]
   Fig. 5 shows the results of IPGTT in the same mice as in Fig. 1 (60 days after Ad vector administration). Blood was collected at the time when glucose (2 g/kg body weight) was administered, 30, 60 and 120 min after administration, and blood glucose level and plasma insulin level were measured. Fig. 5A shows time-course changes in the blood glucose level (mg/dl) after glucose administration, and Fig. 5B shows time-course changes in the plasma insulin level (ng/ml) after glucose administration. *: p<0.05 (with respect to Ad.CA-LacZ); #: p<0.05 (with respect to Intact); n.s.: not significant

### [Description of Embodiments]

The present invention provides a safe and effective agent for protecting and/or regenerating pancreatic β cells in a mammal with diabetes, containing a recombinant viral vector expressing HGF (hereinafter also to be referred to as "the agent for protecting and/or regenerating β cells of the present invention"). The agent for protecting and/or regenerating β cells is characterized in that it is administered at a dose of 10¹⁰ - 10¹² virus particles (vp)/kg body weight, and the viral vector contains a nucleic acid encoding HGF downstream of a promoter with transcriptional activity capable of affording a therapeutically effective blood HGF level at said dose.

In the present specification, "protecting and/or regenerating pancreatic β cells" means that the remaining β cell function is preserved to the extent that compensatory hypersecretion of insulin from β cells does not occurs and normal insulin secretion is maintained, and at least the acute phase of hyperglycemia is significantly suppressed as compared to the control without a therapeutic treatment, and the suppression tendency is maintained over a long term thereafter (e.g., not less than 60 days, preferably not less than 75 days, more preferably not less than 90 days, further preferably not less than 120 days) and/or β cells proliferate. The "protecting and/or regenerating pancreatic β cells" in the present invention inevitably involves "suppression of hyperglycemia", and thus, "the agent for protecting and/or regenerating β cells of the present invention" can also be an "agent for suppressing hyperglycemia". In addition, since suppressing hyperglycemia and controlling blood sugar is most important in treating diabetes and suppressing progression to complications, the "agent for protecting and/or regenerating β cells of the present invention" is also an "agent for treating diabetes".

According to the above-mentioned Non Patent Literature 3, high dose administration of an Ad vector expressing HGF under the control of CMV promoter suppressed hyperglycemia, but significantly increased the blood insulin/glucose ratio, and the authors speculate that it suggests occurrence of compensatory hypersecretion of insulin. Therefore, the agent for protecting and/or regenerating β cells of the present invention capable of suppressing hyperglycemia while maintaining normal insulin secretion, without inducing compensatory hypersecretion of insulin affords advantageous effects such as reduction of the risk of β cell exhaustion and β cell dysfunction due to insulin hypersecretion.

The viral vector used for HGF-expressing recombinant viral vector which is the active ingredient of the agent for protecting and/or regenerating β cells of the present invention is not particularly limited as long as it is generally used for gene therapy. For example, adenovirus (Ad) vector, adeno-associated virus (AAV) vector, lentivirus vector, retrovirus vector, sindbis virus vector, rabies virus vector, Sendaivirus vector, simple herpes virus vector, and the like can be used. It is preferable to use an Ad vector or an AAV vector from the aspects of low frequency of chromosomal integration and no risk of insertional mutation, introducibility into nondividing cells, medium- to long-term expression of transgene, and the like.

The transgene expression period of Ad vector (generally 2-3 weeks) is shorter than that of AAV vectors and chromosomal-integrating vectors. However, since the pancreatic β cells protection effect by HGF persists far beyond the gene expression period (at least 60 days or more, preferably 75 days or more, more preferably 90 days or more, further preferably 120 days or more), it may be rather advantageous in that the risk of side effects due to long-term expression of HGF such as carcinogenesis can be reduced or avoided. Although the size of the gene that the AAV vector can carry is as small as 4.7 kb, since the HGF coding sequence (CDS) is about 2.2 kb, and the entire expression cassette including the promoter, terminator, and so on is about 3 to 4 kb, there is no problem in use thereof.

Ad vector is known to accumulate in the liver. Even if HGF is introduced and expressed in the cells of other organs, it is extracellularly secreted and delivered to the pancreas through the bloodstream. In addition, AAV vectors have different tissue tropism depending on the serotype, and serotypes having tropism toward the pancreas include, for example, types 6, 8, and 9. However, the serotype to be used is not particularly limited, since HGF introduced and expressed in the cells of other organs can still be delivered to the pancreas through the bloodstream when a ubiquitous promoter is used. Rather, in some cases, it may be preferable to express HGF in the cells of other organs because, when viral vectors accumulate in the pancreas, β cells may be attacked by viral capsid antigen-specific killer T cells.

The HGF expressing recombinant viral vector used in the present invention characteristically contains a nucleic acid encoding HGF downstream of a promoter with transcriptional activity capable of affording a therapeutically effective blood HGF level when administered at a dose of 10¹⁰ - 10¹²vp/kg body weight. Here, the "therapeutically effective blood HGF level" means a blood HGF level sufficient to afford the aforementioned effect of "protecting and/or regenerating pancreatic β cells". The blood HGF level is not particularly limited as long as it is within a concentration range that affords the effect of protecting and/or regenerating pancreatic β cells in mammals with diabetes. For example, the peak blood HGF level may be not less than 2 ng/ml, preferably 2 to 5 ng/ml, and the average blood HGF level for one week after administration may be not less than 0.6 ng/ml, preferably not less than 1 ng/ml.

The "promoter with transcriptional activity capable of affording a therapeutically effective blood HGF level" is not particularly limited as long as it has a level of transcriptional activity that can achieve the above-mentioned blood HGF level when administered at a dose of 10¹⁰ - 10¹² vp/kg body weight. In various cell types and depending on the type of viral vector to be used, promoters with stronger transcriptional activity than the CMV promoters used in existing HGF gene therapy studies for diabetes can be used. As such high active promoter, CA promoters and promoters with transcriptional activity equivalent thereto, for example, ubiquitous promoters such as polypeptide chain elongation factor 1α1 (EF1A) promoter, polypeptide chain elongation factor 1α1 short (EFS) promoter, CBh promoter (hybrid promoter of CMV immediate-early enhancer and modified chicken β-actin promoter different CA promoter), spleen focus-forming virus (SFFV) promoter, mouse stem cell virus (MSCV) promoter, simian virus 40 (SV40) enhancer/early promoter, phosphoglycerate kinase (PGK) promoter, ubiquitin C (UBC) promoter, and the like can be mentioned. In addition, when a high viral vector with high tissue tropism is used, or in case of topical administration, a promoter which is specifically and highly expressed in tissue or cells of the target organ (e.g., in liver; albumin promoter, α-fetoprotein promoter, thyroxine-binding globulin promoter, and the like, in pancreatic β cells, insulin promoter, Pdx1 promoter, Ins2 promoter, and the like, in muscle; myogenin promoter, skeletal muscle actin α1 (ACTA1) promoter, MHCK7 promoter, SM22a promoter, and the like can be unlimitatively mentioned, including promoters specific for tissues or cells of any organ from which the secreted and expressed HGF can be delivered to the pancreas by the bloodstream) can also be used. That the promoter to be used has transcriptional activity capable of affording a therapeutically effective blood HGF level can be confirmed by, for example, administering a viral vector containing a nucleic acid encoding HGF downstream of the promoter to experimental animals such as mouse at a dose of 10¹⁰ - 10¹² vp/kg body weight, measuring the HGF levels in blood collected over time after administration by ELISA and the like, and examining that it is at the above-mentioned therapeutically effective level. More conveniently, it can also be confirmed by, for example, infecting a panel of cultured human cells with a viral vector into which a reporter gene such as GFP is inserted instead of HGF, and measuring the transcriptional activity for CMV promoter by using the reporter activity as an index.

In a particularly preferred embodiment, CA promoter can be mentioned as the promoter that drives HGF expression. The CA promoter used in the present invention is a nucleic acid consisting of the nucleotide sequence shown by SEQ ID NO: 1 or a nucleic acids capable of hybridizing to a complementary strand sequence of the nucleic acid under stringent conditions, and includes a nucleic acid having transcriptional activity equal to or higher than that of the nucleic acid consisting of the nucleotide sequence shown by SEQ ID NO: 1. As such nucleic acid, a nucleic acid containing a nucleotide sequence having an identity of about 80% or more, preferably about 90% or more, more preferably about 95% or more, particularly preferably about 97% or more, most preferably about 98% or more, with the nucleotide sequence shown by SEQ ID NO: 1, and the like can be mentioned. The homology of the nucleotide sequence in the present specification can be, for example, calculated using homology calculation algorithm NCBI BLAST (National Center for Biotechnology Information Basic Local Alignment Search Tool) under the following conditions (expectancy=10; allowing gap; filtering=ON; match score=1; mismatch score=-3).

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under highly stringent conditions. The stringent conditions are exemplified by reaction conditions characterized in that (1) a low ionic strength and a high temperature, for example, 0.015 M sodium chloride/0.0015 M sodium citrate/0.1% dodecyl sodium sulfate at 50°C, is used for washing, and (2) a denaturing agent such as formamide, for example, 50% (v/v) formamide along with a 50 mM sodium phosphate buffer (pH 6.5) containing 0.1% bovine serum albumin/0.1% Ficoll/0.1% polyvinylpyrrolidone/750 mM sodium chloride and 75 mM sodium citrate is used at 42°C. Alternatively, the stringent condition can be a condition in which 50% formamide, 5xSSC (0.75 M NaCl, 0.075 M sodium citrate), 50 mM sodium phosphate (pH 6.8), 0.1% sodium pyrophosphate, 5x Denhart's solution, sonicated salmon sperm DNA (50 µg/ml), 0.1% SDS, and 10% dextran sulfate are used at 42°C, and a washing is performed with 0.2xSSC and 50% formaldehyde at 55°C, followed by a high-stringent washing comprised of EDTA-containing 0.1xSSC at 55°C. Those of ordinary skill in the art can easily achieve a desired stringency by appropriately adjusting temperature at hybridization reaction and/or washing, ion strength of buffer, and the like based on factors such as probe length.

As the "nucleic acid encoding HGF" used in the present invention, a nucleic acid encoding a protein containing the nucleotide sequence shown by SEQ ID NO:2 (corresponding to nucleotide sequence (CDS) from positions 77 to 2263 of human HGF mRNA sequence registered in GenBank under Accession Number: NM_000601), or a nucleotide sequence that hybridizes to a complementary strand sequence thereof under stringent conditions, and having activity (e.g., pancreatic β cell protecting and/or regenerating activity) equivalent to that of HGF can be mentioned.

Examples of the nucleic acid that hybridizes to the complementary strand sequence of the nucleotide sequence shown by SEQ ID NO:2 under stringent conditions include a nucleic acid containing a nucleotide sequence showing an identity of about 60% or more, preferably about 70% or more, more preferably about 80% or more, and particularly preferably about 90% or more, most preferably about 95% or more, with the nucleotide sequence shown by SEQ ID NO:2, and the like. As used herein, the "stringent conditions" are as defined for the aforementioned promoters. The nucleic acid encodes an amino acid sequence showing an identity of about 90% or more, preferably about 95% or more, further preferably about 97% or more, and particularly preferably about 98% or more, with the amino acid sequence shown by SEQ ID NO:3, such that a protein containing the amino acid sequence has substantially the same activity (e.g., pancreatic β cell protecting and/or regenerating activity) as a protein containing the amino acid sequence shown by SEQ ID NO:3.

The nucleic acid encoding HGF may be an ortholog, in non-human mammals, of the nucleic acid consisting of the nucleotide sequence shown by SEQ ID NO:2. For example, it is desirable to use a nucleic acid encoding HGF derived from a mammal as a subject of administration. The mammal to be the subject of administration of the agent for protecting and/or regenerating β cells of the present invention is not particularly limited as long as it has diabetes, and includes human, mouse, rat, rabbit, dog, monkey, and the like, preferably human. Therefore, in a preferred embodiment, the nucleic acid encoding HGF is a nucleic acid encoding human HGF (i.e., a protein consisting of the amino acid sequence shown by SEQ ID NO:3).

A nucleic acid encoding HGF can be cloned, for example, by amplifying same by the PCR method using a synthetic DNA primer containing a portion of the nucleotide sequence of the CDS region of HGF gene, or by hybridizing a DNA incorporated in an appropriate expression vector to a DNA fragment containing the nucleotide sequence of the CDS region of the HGF gene or one labeled with a synthetic DNA. Hybridization can be conducted according to, for example, a method described in Molecular Cloning, 2nd edition (ibidem) and the like. When a commercially available library is used, hybridization can be conducted according to the method described in the instruction manual attached to the library.

The nucleotide sequence of DNA can be converted according to a method known per se, such as the ODA-LA PCR method, the Gapped duplex method, the Kunkel method and the like, or a method based thereon, using a publicly known kit, for example, Mutan^{™}-super Express Km (Takara Shuzo Co., Ltd.), Mutan^{™}-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

An expression vector containing a nucleic acid encoding HGF can be produced, for example, by cutting out a desired fragment from the nucleic acid encoding CDS region of the HGF gene, and linking the fragment downstream of a promoter in the above-mentioned expression vector. The expression vector preferably contains a transcription termination signal, i.e., terminator region, in the downstream of the nucleic acid encoding HGF. The expression vector can further contain a selection marker gene for selection of transformed cells (genes that offer resistance against agents such as tetracycline, ampicillin, kanamycin, hygromycin, and phosphinothricin, genes that complement an auxotrophic mutation, and the like).

The HGF expression recombinant viral vector of the present invention can be produced by using a conventional genetic engineering technique, cell culturing technique and virus preparation technique [for example, Current Protocols in Molecular Biology, F. Ausubel et al. eds. (1994) John Wiley & Sons, Inc.; Molecular Cloning (A Laboratory Manual), 3rd ed. Volumes 1-3, Josseph Sambrook & David W. Russel eds., Cold Spring Harbor Laboratory Press (Cold Spring Harbor, New York) (2001); Culture of Animal Cells; A Manual of Basic Technique, R. Freshney eds., 2nd ed. (1987), Wiley-Liss; Frank L. Graham, Manipulation of adenovirus vector, Chapter 11. p109-p128; E.J. Murray eds., Methods in Molecular Biology, Vol. 7, Gene Transfer and Expression Protocols (1991); Chen, S-H. et al., Combination gene therapy for liver metastases of colon carcinoma in vivo., Proc. Natl. Acad. Sci. USA (1995) 92, 2477-2581, and the like].

The agent for protecting and/or regenerating β cells of the present invention can suppress hyperglycemia over a long period of time while maintaining normal insulin secretion even when administered at a low dose that does not substantially cause adverse events due to viral vector. Therefore, it can be used for the treatment of T1D and other diabetes (e.g., T2D where insulin resistance progresses and β cells are exhausted due to compensatory insulin hypersecretion, resulting in impaired insulin secretion and eventual β cell death) requiring insulin administration due to the destruction of pancreatic β cells, as well as for the suppression of the progression into complications.

In the agent for protecting and/or regenerating β cells of the present invention, the HGF-expressing recombinant viral vector of the present invention may be used as it is, or may be, where necessary, mixed with a pharmacologically acceptable carrier and formulated into various forms of preparation such as injection and the like, and used as a pharmaceutical agent.

Here, as examples of the pharmacologically acceptable carrier, various organic or inorganic carrier substances conventionally used as pharmaceutical preparation materials can be mentioned, and in liquid preparations, these are formulated as solvents, solubilizing agents, suspending agents, isotonizing agents, buffering agents and soothing agents, and the like. Also, as necessary, pharmaceutical preparation additives such as antiseptics, antioxidants, colorants, and the like can be used.

As examples of suitable solvents, water for injection, physiological saline, Ringer's solutions, alcohols, propylene glycol, polyethylene glycol, sesame oil, corn oil, olive oil, cottonseed oil and the like can be mentioned.

As examples of suitable solubilizing agents, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of suitable suspending agents, surfactants such as stearyl triethanolamine, sodium lauryl sulfate, lauryl aminopropionic acid, lecithin, benzalkonium chloride, benzethonium chloride and glyceryl monostearate; hydrophilic polymers such as polyvinyl alcohol, polyvinyl pyrrolidone, sodium carboxymethyl cellulose, methyl cellulose, hydroxymethyl cellulose, hydroxyethyl cellulose and hydroxypropyl cellulose; polysorbates, polyoxyethylene hardened castor oil and the like can be mentioned.

As examples of suitable isotonizing agents, sodium chloride, glycerin, D-mannitol, D-sorbitol, glucose and the like can be mentioned.

As examples of suitable buffers, buffer solutions of a phosphate, an acetate, a carbonate, a citrate and the like, and the like can be mentioned.

As examples of suitable soothing agents, benzyl alcohol and the like can be mentioned.

As examples of suitable antiseptics, paraoxybenzoates, chlorobutanol, benzyl alcohol, phenethyl alcohol, dehydroacetic acid, sorbic acid and the like can be mentioned.

As examples of suitable antioxidants, sulfides, ascorbates and the like can be mentioned.

As examples of suitable colorants, aqueous food tar colors (e.g., food colors such as Food Red Nos. 2 and 3, Food Yellow Nos. 4 and 5, and Food Blue Nos. 1 and 2), water-insoluble lake pigments (e.g., aluminum salts of the aforementioned aqueous food tar colors and the like), natural pigments (e.g., β-carotene, chlorophyll, red iron oxide and the like) and the like can be mentioned.

As examples of dosage forms of the aforementioned pharmaceutical composition, parenteral formulations such as injections (e.g., subcutaneous injections, intravenous injections, intramuscular injections, intraperitoneal injections and the like), drip infusion and the like can be mentioned.

The agent for protecting and/or regenerating β cells of the present invention can be produced by a method conventionally used in the field of formulation technology, such as the method described in the Japanese Pharmacopoeia. The content of the viral vector, which is the active ingredient in the preparation, varies depending on the dosage form, dose of the active ingredient, and the like and is, for example, about 0.1 to 100 wt%. The virus titer can be appropriately adjusted to be about, for example, 10¹⁰ - 10¹¹ pfu/ml (since the physical titer is several to 100 times or more higher than the biological titer, 2×10¹⁰ - 2×10¹³ vp/ml in terms of virus particles), but is not limited to this range.

Examples of preparations suitable for parenteral administration (e.g., intravenous injection, subcutaneous injection, intramuscular injection, topical injection, intraperitoneal administration and the like) include aqueous and nonaqueous isotonic aseptic injection liquids, in which antioxidant, buffer, antibacterial agent, isotonicity agent and the like can be contained. The examples also include aqueous and nonaqueous aseptic suspension liquids optionally containing suspension agent, solubilizer, thickener, stabilizer, preservative and the like. The most preferred dosage form in the present invention is injection liquid.

The agent for protecting and/or regenerating β cells of the present invention is characterized in that it is administered to human or other mammalian subjects at a dose of 10¹⁰ - 10¹² vp/kg body weight, which is lower than the existing HGF gene therapy for diabetes (5×10¹² to 1.5×10¹³ vp/kg body weight). By administering at such a low dose, the agent for protecting and/or regenerating β cells of the present invention can be used safely without substantially causing adverse events (particularly liver dysfunction characterized by increased AST and ALT levels). Moreover, since the expression of HGF is driven by a promoter with strong transcriptional activity, even administration of such a low dose can achieve a therapeutically effective blood HGF level, and sufficient pancreatic β cell protecting and/or regenerating effects can be obtained.

The dose of the preparation can be appropriately selected within the above-mentioned range, according to the factors of the preparation itself such as the type of vector, promoter activity, viral vector infectivity (physical titer: biological titer (vp: PFU) ratio, and the like, and external factors such as administration route, severity of illness, the animal species to be the subject of administration, drug acceptability, body weight, age and the like of the subject of administration. Particularly for Ad vector, the vp:PFU ratio is an important parameter for determining the viral dose, since the viral particles themselves can induce dose-dependent acute toxicity. For example, in the below-mentioned Example, administration of 3×10⁸ PFU/mouse (body weight about 20 g; about 1.5×10¹⁰ PFU per 1 kg) of Ad vector from the tail vein was confirmed to afford the effect of protecting and/or regenerating pancreatic β cells (suppression of hyperglycemia and normal insulin secretion). However, since the vp:PFU ratio varies greatly in the range of several times to 100 times or more due to the influence of the virus extraction method and the like, the dose based on the physical titer may be about 3×10¹⁰ - about 3×10¹² vp/kg body weight. However, as mentioned above, hepatic artery administration of 6×10¹¹ vp/kg body weight of Ad vector was reported to have caused death due to acute liver injury (the above-mentioned Non Patent Literature 4). When using an Ad vector, it is desirable to administer same at a dose of 10¹¹ vp/kg body weight or less. However, in the case of hepatic artery administration as in the Literature, a higher dose of viral vector is considered to accumulate in the liver. For other systemic administrations, particularly when viral vector is administered from a peripheral vein, it is assumed that even a dose of 10¹² vp/kg body weight can be safely used. On the other hand, in the case of AAV vectors, since no adverse events in the liver occurred in the low dose (1×10¹⁴ vp/kg body weight) administration group despite the presence of hepatic diseases, even in the recent clinical trials in which deaths due to severe liver damage were reported in the high-dose (3×10¹⁴ vp/kg body weight) administration group, it is assumed that the dose of 10¹² vp/kg body weight can be used safely.

Note that the U.S. Food and Drug Administration (FDA) recommends a vp:PFU ratio of less than 30 for clinical-grade Ad vectors. When Ad vector produced for clinical application is injected from peripheral veins at an infection titer of 1.5×10¹⁰ PFU/kg body weight, it would be administered at a dose of less than 4.5×10¹¹ vp/kg body weight. Thus, it is considered a sufficiently low dose compared to the hepatic artery administration at 6×10¹¹ vp/kg body weight.

The agent for protecting and/or regenerating β cells of the present invention is preferably administered, for example, parenterally (e.g., intravenously, subcutaneously, intramuscularly, intraperitoneally, topical injecting, and the like) by injection, catheter, balloon catheter, or the like, and systemic administration (e.g., intravenous, intraarterial, intramuscular, intraperitoneal administration, etc.) is more preferred. Local administration into the pancreatic duct is considered to be apparently advantageous in that the risk of adverse events due to gene transfer to multiple organs can be avoided, since gene transfer is almost confined to the pancreas. However, given the risk that β cells may be destroyed by the attack of killer T-cells against transgenes and viruses, systemic administration, which allows for gene transfer to other organs, is clinically advantageous. Among the dose ranges of the viral vector used in the present invention, when a comparatively high dose of 5×10¹¹ - 1×10¹² vp/kg body weight is used, in order to avoid liver damage, which is particularly important as an adverse event caused by the viral vector, it is desirable to avoid administration routes such as hepatic arterial administration that deliver high doses of the viral vector to the liver, and administer the agent for protecting and/or regenerating β cells of the present invention from, for example, peripheral vein or the like.

The administration frequency of the agent for protecting and/or regenerating β cells of the present invention is not particularly limited. As shown in the following Example, even when an Ad vector is used, a single administration can afford a certain degree of hyperglycemia suppressive effect over a long period of at least about 2.5 months. In addition, the glucose-responsive insulin secretory capacity of pancreatic β cells can be maintained (improved glucose tolerance) for at least 60 days. The mouse of Example is under ongoing progress observation, and it is fully expected that the above-mentioned effects will continue for an even longer period of time. The same may be true when an AAV vector, the transgene of which is in principle not integrated into the chromosome, is introduced into dividing cells.

In addition, when the AAV vector is used to target nondividing cells, HGF expression can be maintained for a longer period of time. Therefore, it is considered that a hyperglycemia suppressive action and a glucose tolerance improving effect can be afforded for even longer period of time (e.g., 6 months or longer, preferably one year or longer, more preferably several years or longer).

Therefore, the agent for protecting and/or regenerating β cells of the present invention can be administered at intervals of, for example, at least 60 days or longer, preferably 75 days or longer, more preferably 90 days or longer, and further preferably 120 days or longer, even when Ad vectors and AAV vectors, which are non-chromosomally integrated and conventionally considered safer and used more frequently than retrovirus and lentivirus vectors, are used. In addition, depending on the type of viral vector to be used, it can also be administered at an interval of once every three months to several years, and in another embodiment, single administration is also possible.

The present invention is explained in more detail in the following by referring to Examples, which are mere exemplifications and do not limit the scope of the present invention in any manner.

### [Example]

### Experiment method

### 1. Production of recombinant adenovirus vector (Ad)

Non-proliferative Ad that expresses human HGF under transcriptional control of a hybrid promoter (CA promoter) of a cytomegalovirus immediate early enhancer and an altered chicken β-actin promoter (to be referred to as "Ad.CA-HGF" in the present specification), and non-proliferative Ad that similarly expresses LacZ gene under transcriptional control of CA promoter (to be referred to as "Ad.CA-LacZ" in the present specification) were produced by the method described in HUMAN GENE THERAPY 10:2013-2017 (1999). The thus-produced recombinant Ads were proliferated, purified, and the titer was measured by a conventional method described in Proc. Natl. Acad. Sci. USA 92: 2577-2581 (1995).

### 2. Animal experiment

Male 8-week-old c57/BL/6N mice (Kyudo Co., Ltd., Tosu) weighing 18-20 g were bred with free access to feed and water. As previously reported (Diabetes 2010; 59: 1261-1265), streptozotocin (STZ; Sigma-Aldrich Japan, Tokyo) dissolved in 0.01 M citrate buffer (pH 4.5) was intraperitoneally administered once per day at a dose of 50 mg/kg for 5 consecutive days (from day -7 to day -3) to 25 mice. The mice injected with STZ were randomly divided into two groups three days later (day 0) and 3×10⁸ plaque-forming unit (pfu) of Ad.CA-LacZ (n=13) or Ad.CA-HGF (n=12) was injected once from the tail vein. Mice (n=8) that did not receive STZ injection or adenoviral gene therapy were used as normal control. Blood was collected from all mice, including those in the normal group. Blood was collected daily from day -7 to day 7, and once a week from day 14 to day 77. Blood glucose, plasma insulin, and plasma aspartate aminotransferase (AST) levels were tested at the time points indicated in each Figure. Intraperitoneal glucose tolerance test (IPGTT) was performed 16 and 60 days after Ad vector administration. After fasting the mice for 14 hr, 2 g/kg body weight of glucose (equivalent to 75 g oral glucose tolerance test (OGTT) in human) was administered intraperitoneally, blood was collected immediately before administration, and 30, 60, and 120 min after administration, and blood glucose level and plasma insulin level were measured at each time point. Insulin was measured using a Morinaga Ultra Sensitive Mouse/Rat Insulin ELISA kit (Morinaga Institute of Biological Science).

All animal experiments were conducted in accordance with the guidelines of the National Institutes of Health, and were approved by the Kagoshima University Animal Experiment Ethics Committee.

### 3. Biochemical analysis

Blood glucose level was measured using Glucocard G Black (Arkray, Kyoto) and Medisafefit Pro II (Terumo, Tokyo), and plasma AST and ALT levels were measured using a SPOTCHEM SP-4430 clinical automatic monitor (Arkray). Plasma insulin level was measured by ELISA assay (Morinaga, Yokohama).

### 4. Statistical analysis

Data are presented as mean±standard error (s.e.). Multiple comparisons were tested by one-way ANOVA. The presence or absence of statistically significant difference between Ad.CA-LacZ-treated mice and Ad.CA-HGF-treated mice was determined by Student's t-test. P<0.05 was defined as statistically significant.

### Experiment results

### 1. Suppression of acute-phase hyperglycemia in T1D model mouse by low-dose Ad.CA-HGF gene therapy

In the non-treated group (Ad.CA-LacZ administration group), the blood glucose concentration rapidly increased to 250 mg/dl on the 7th day after vector administration (14 days from the first STZ injection) (Fig. 1A), then continued to rise gradually thereafter, and remained at a high level of around 270 mg/dl (Fig. 1B). When 3×10⁸ pfu (corresponding to about 10⁹ - about 10¹⁰ vp for clinical grade Ad vectors) of Ad.CA-HGF was intravenously injected once, the increase in blood glucose concentration between days 3 and 6 was significantly suppressed (Fig. 1A). After about 3 months of progress observation, the blood glucose level showed a tendency of being suppressed as compared with the non-treated group, and a long-term effect was observed (Fig. 1B). Animal experiment was conducted again using the same protocol as above, and similar results were obtained, confirming the reproducibility of the hyperglycemia suppressing effect by the low dose HGF administration (Figs. 1C, 1D) .

In a past report (Exp Mol Med 2003; 35: 494-500), Ad vector expressing HGF under the control of CMV promoter was administered at a high dose of 1×10¹¹ vp (about 5×10¹² vp/kg body weight) which is one or two orders of magnitude higher than that assumed in this Example. Although simple comparison cannot be made because the administration routes are different, this dose is nearly 10 times higher than that in the case of death due to the Ad vector (the above-mentioned Non Patent Literature 4), which has prevented its application to human clinical practice. The present invention is extremely significant in that it has succeeded in affording a hyperglycemia suppressing effect the same as or higher than that in previous reports even at a dose as low as 1/10 to 1/100 that in the previous reports, by using a CA promoter with a stronger transcriptional activity.

### 2. Suppression of acute plasma insulin elevation in T1D model mice by low-dose Ad.CA-HGF gene therapy (protection and/or regeneration action on remaining β cells)

In order to evaluate the mechanism by which the therapeutic effect is obtained, the plasma insulin concentration on day 7, day 14, and day 21 was measured in the STZ-injected mice of the above-mentioned 1 (Fig. 2). On day 7, a remarkable increase in the plasma insulin concentration was observed in the non-treated group (Ad.CA-LacZ administration group). This phenomenon is consistent with the previous knowledge that β-cells are destroyed by STZ treatment, and the remaining β-cells compensatorily highly produce and/or secrete insulin temporarily and abnormally (Environ Toxicol Pharmacol 2001; 9: 71-78), and suggests that β-cell death is progressing in the control mice. On the other hand, in the Ad.CA-HGF administration group, no increase in plasma insulin concentration was observed on day 7, and the plasma insulin level remained normal thereafter. These results suggest that, due to the systemic administration of low doses of Ad.CA-HGF, HGF secreted from transfected cells was delivered to the pancreas through the bloodstream and suppressed cell death of β cells (cell protection action), allowing a greater number of β-cells to remain, and a therapeutic effect (hyperglycemia suppressing effect) was provided while maintaining normal insulin secretion. This provides a further superiority to the previous report (Exp Mol Med 2003; 35: 494-500) in which high insulin/glucose ratio was observed, suggesting that administration of high dose HGF-expressing Ad vector induced compensatory hypersecretion of insulin.

### 3. Intravenously administration of low dose Ad vector does not cause liver dysfunction

In preclinical and clinical test studies, it was reported that intravenous administration of high doses of Ad vectors in vivo resulted in gene transfer primarily into the liver, which could lead to severe liver injury (Mol Genet Metab 2003; 80: 148-158.; Hum Gene Ther 2020; 31: 695-696). Therefore, in order to evaluate liver injury due to intravenous administration of a low dose Ad vector, plasma AST and ALT levels were measured on day 7, day 14, and day 21 in each group of mice in the above-mentioned 1. No significant increase in the plasma AST level was observed by week 3 in any of the mice that received Ad vector administration (Ad.CA-LacZ administration group and Ad.CA-HGF administration group) (Fig. 3). Similarly, no significant increase in the plasma ALT level was observed up to 3 weeks. During the subsequent progress observation period, all the mice remained in good health with no change in appetite and movement, and survived until day 77 when the experiment was terminated. These data suggest that a gene therapy strategy of a single intravenous injection of a low dose of an Ad vector expressing the HGF gene under the transcriptional control of a strong promoter is effective and safe.

### 4. Intravenously administration of low dose Ad vector improves glucose tolerance in T1D model mouse

IPGTT was performed 16 days and 60 days after administration of the Ad vector, and suppression of postprandial hyperglycemia and glucose-responsive insulin secretion capacity were confirmed. In the HGF gene administration group, suppression of blood glucose elevation after glucose administration was observed on both day 16 (Fig. 4) and day 60 (Fig. 5) of administration (Figs. 4A and 5A), and an increase in the secretion amount was confirmed in insulin secretion reaction in response to glucose stimulation (Figs. 4B, 5B) .

### [Industrial Applicability]

Recombinant viral vector that expresses HGF under the transcriptional control of a strong promoter such as CA promoter shows an effect of protecting and/or regenerating pancreatic β cells by administration at low doses, and affords hyperglycemia-suppressing effects while maintaining normal insulin secretion. Therefore, it can avoid the risk of adverse events associated with administration of high doses of viral vector, and furthermore, the effect is maintained for a long period of time far exceeding the expected gene expression period even when a chromosomally non-integrating virus vector, which has conventionally been considered relatively safe, is used. Therefore, the agent for protecting and/or regenerating β cells of the present invention can be a gene therapy agent for diabetes that is safe and effective and can be clinically applied. The number of diabetes patients continues to increase worldwide and has become a social problem, and the significance of the present invention is high. In particular, T1D develops at a young age, and existing pancreatic islet transplantation therapy is limited in use. Therefore, the agent for protecting and/or regenerating pancreatic β cells of the present invention is extremely useful as an alternative and versatile means of regenerative medicine for diabetes including T1D.

This application is based on a patent application No. 2020-192844 filed in Japan (filing date: November 19, 2020) and a patent application No. 2021-145795 filed in Japan (filing date: September 7, 2021), the contents of which are incorporated in full herein.

## Claims

1. An agent for protecting and/or regenerating pancreatic β cells in a mammal with diabetes, comprising a recombinant viral vector expressing a hepatocyte growth factor (HGF), wherein the agent is administered at a dose of 10¹⁰ - 10¹² virus particles (vp)/kg body weight, and the viral vector comprises a nucleic acid encoding HGF downstream of a promoter with transcriptional activity capable of affording a therapeutically effective blood HGF level at said dose.

2. The agent according to claim 1, wherein the viral vector is an adenovirus (Ad) vector or an adeno-associated virus (AAV) vector.

3. The agent according to claim 2, wherein the agent is administered in a single dose or multiple doses at an administration interval of at least 60 days.

4. The agent according to any one of claims 1 to 3, wherein the promoter is a CA promoter.

5. The agent according to any one of claims 1 to 4, wherein the diabetes is type 1 diabetes.

6. The agent according to any one of claims 1 to 5, wherein the mammal is human.

7. The agent according to any one of claims 1 to 6, wherein the agent is administered by systemic administration.

8. The agent according to claim 7, wherein the systemic administration is intravenous administration.

9. The agent according to claim 8, wherein the intravenous administration is administration via a peripheral vein.

10. A method for protecting and/or regenerating pancreatic β cells in a mammal with diabetes, comprising administering a recombinant viral vector expressing HGF to the mammal, wherein the viral vector is administered at a dose of 10¹⁰ - 10¹² virus particles (vp)/kg body weight, and comprises a nucleic acid encoding HGF downstream of a promoter with transcriptional activity capable of affording a therapeutically effective blood HGF level at said dose.
